# EUROPÄISCHE PATENTSCHRIFT

(11) **EP 0 891 777 B1**
(45) Veröffentlichungstag und Bekanntmachung des Hinweises auf die Patenterteilung: **24.01.2001**
(21) Anmeldenummer: 98106029.6
(22) Anmeldetag: 02.04.1998
(51) Int. Cl.: A61K 35/02, A61K 35/78

(54) **Packung auf Basis von Fango und Verfahren zur Herstellung derselben**
Fango containing pack and process for producing the same
Pack au fango ainsi que son procédé de production

(30) Priorität: 21.06.1997 DE 19726366
(43) Veröffentlichungstag der Anmeldung: 20.01.1999
(73) Patentinhaber: Aktiengesellschaft Bad Neuenahr, 53474 Bad Neuenahr-Ahrweiler (DE)
(72) Erfinder: Mertel, Rainer, 53498 Bad Breisig (DE)
(74) Vertreter: Werner, Hans-Karsten, Dr.Dipl.-Chem.

(56) Entgegenhaltungen:
- DE-A- 19 541 735

## Beschreibung

Gegenstand der vorliegenden Erfindung ist eine Packung auf Basis von Fango. Fango sind vulkanische Erden mit hohem Gehalt an Kieselsäuren und Aluminiumoxid sowie einer Reihe von weiteren mineralischen Bestandteilen und Spurenelementen.

Fango ist im Gegensatz zum Moor ein anorganisches Naturprodukt. Fangopackungen werden im allgemeinen in der Weise angewendet, daß das feuchte und knetbare Material erhitzt als sogenannte Wärmepackung oder kalt auf den erkrankten Körperteil aufgestrichen wird. Der Patient wird mit einem Leinen-Nessel-Tuch eingepackt sowie mit einer Wolldecke.

Sofern bei der Anwendung nur die im Gestein gespeicherte Wärme gewünscht wird, können Gemische aus Vulkangestein und Wachs zusätzlich mit einem Wärmespeichergranulat und einer Hülle aus Kunststoff versehen werden. Diese Packungen können weiterhin mit einem Stoffbeutel überzogen werden, auf den vor der Anwendung ätherische Öle und/oder Arzneimittelwirkstoffe aufgetragen werden können; vgl. DE-U-295 02 489. Die Heilwirkung des Fango kommt hierbei aber nicht zum Tragen, da der Pflanzenextrakt und/oder das ätherische Öl lediglich oberflächlich auf einen Stoff aufgetragen, mit der menschlichen Haut in Berührung kommt. Dem Trägerstoff gemäß Stand der Technik kommt somit ausschließlich wärmespeichernde Wirkung zu.

Die DE-A- 195 41 735 beschreibt die Verwendung hydrothermaler Gesteinsablagerungen zur Verbesserung der Lichtquanten-Resonanzeffekte im Körper durch molekulardisperse Verteilung insbesondere zur Gesundheitsvorsorge durch Thermal-, Heil-, Sole-, Fango-, Schwefel- und Mineralvorkommen, ihre ganzheitliche Rückverwandlung bis zu einer molekulardispersen Verteilung, ganzheitlich homogenisiert mit Edelkristallen, pflanzlichen und tierischen Stoffen als kolloidale Verbundstoffe, zur verbesserten Steuerung der Biophotonenstrahlung durch Nanokristalle etc., wobei auch hydrothermale Ablagerungen aufgezählt sind.

Noch weniger wirksam sind geschlossene Taschen aus Plastikfilm gemäß FR-A-2 721 205, in die Wachs, Vulkanerde und gegebenenfalls ätherische Öle eingebracht werden, da hier die ätherischen Öle keinen Kontakt mit der Haut haben und deshalb auch nicht in die Haut eindringen können.

Die Sorptionskraft des Eifelfangos (Eifelfango-Chemisch-Pharmazeutische-Werke in Bad Neuenahr) bindet aber bei direktem Hautkontakt die während des Pakungsverlaufs erfolgende Körperausscheidung, so daß - anders als bei Moor und Parafingemischen, die bekanntlich mehrfach verwendet werden - Eifelfango-Packungen aus hygienischen Gründen stets nur einmal zu verwenden sind. Die Fangopackung bleibt ca. 30 Minuten auf dem Körperteil. Nach der Abnahme der Packung wird der Patient geduscht.

Wärmepackungen auf Basis von Fango werden beispielsweise eingesetzt bei chirurgischen und orthopädischen Behandlungen wie Muskel- und Gelenkrheuma, Ischias, postoperativen Behandlungen und Sportverletzungen, bei inneren Erkrankungen wie Magen-, Leber- und Gallenleiden, bei urologischen Erkrankungen, insbesondere Spasmen im Bereich des Urogenitaltraktes, gynäkologischen Erkrankungen wie chronische Endometritis, Salpingitis, Oopheritis und Parametritis, beim rheumatischen Formenkreis wie entzündlichen und degenerativen Formen des Rheumatismus sowie neurologischen Erkrankungen wie Neuralgien und Polyneutritis. Einige der oben genannten Erkrankungen werden auch mit Pflanzenextrakten und/oder ätherischen Ölen behandelt, die entweder als Einreibungen oder in Form von feuchten Umschlägen zur Anwendung kommen. Typische Pflanzenextrakte und/oder ätherische Öle sind die von Arnika, Rosmarin, Kamille, Roßkastanie, Lavendel oder Gemische derselben.

Die Erfindung hat sich die Aufgabe gestellt, die Wirksamkeit von Packungen auf Basis von Fango einerseits und von Pflanzenextrakten und/oder ätherischen Ölen andererseits zu verbessern.

Diese Aufgabe wird in hervorragender Weise und mit synergistischem Effekt gelöst durch eine Packung auf Basis von Fango und einem oder mehreren Pflanzenextrakten und/oder ätherischen Ölen, bei dem Fango und Pflanzenextrakte und/oder ätherische Öle in vermischter Form vorliegen. Die Herstellung einer derartigen Wärmepackung erfolgt dadurch, dass der gegebenenfalls bereits erwärmte, feuchte Fango mit dem oder den Pflanzenextrakten und/oder ätherischen Ölen vermischt und dann in üblicher Weise auf den Körper oder den Körperteil aufgebracht wird.

Das Aufbringen der Pflanzenextrakte und/oder ätherischen Öle in Form der bereits erwärmten Wärmepackung auf Basis von Fango führt zu einer deutlichen Steigerung der Wirksamkeit der Pflanzenextrakte und/oder ätherischen Öle, da diese Applikationsform dafür sorgt, daß die Wirkstoffe der Pflanzenextrakte und/oder ätherischen Öle überwiegend an die Körperoberfläche abgegeben werden, wo sie durch die feuchten und warmen Bedingungen besser in die Haut und durch die Haut eindringen können. Eine erfindungsgemäße Wärmepackung auf Basis von Fango führt somit zu einer besseren und längeranhaltenden Wirkung der Pflanzenextrakte und/oder ätherischen Öle. Die Wirksamkeit der Pflanzenextrakte und/oder ätherischen Öle wird obendrein verstärkt durch die eigentliche Wärmepackung auf Basis von Fango, die ja bereits für sich alleine eine gute Wirksamkeit entfaltet.

Die erfindungsgemäße Packung auf Basis von Fango und das Verfahren zur Herstellung derselben ermöglicht somit eine nicht vorhersehbare Wirkungssteigerung und optimale Ausnutzung der Wirkstoffe der Pflanzenextrakte und/oder ätherischen Öle. Dies gilt sowohl für einzelne Pflanzenextrakte und ätherische Öle als auch für Gemische eines oder mehrerer Pflanzenextrakte und/oder ätherische Öle.

Ein typisches Beispiel für die erfindungsgemäße Wärmepackung auf Basis von Fango ist die Kombination von Arnikaextrakt bei rheumatischen Beschwerden, chronischen Gelenkbeschwerden und Neuralgie. Die Wirksamkeit kann durch eine weitere Kombination mit Rosmarinextrakt gesteigert werden und bei niedrigeren Temperaturen auch bei Krampfadern, Wunden und Verstauchungen zum Einsatz kommen. Bei dieser Anwendung kann sich die zusätzliche Kombination mit Kamilleextrakt empfehlen.

Die Kombination mit Roßkastanienextrakt empfiehlt sich bei niedrigeren Temperaturen bei Krampfadern und Schwellungen sowie Entzündungen. Auch hier kann Kamilleextrakt zusätzlich eingesetzt werden.

Kamilleextrakt alleine in der Wärmepackung eignet sich warm bei Magen- und Verdauungsbeschwerden sowie bei Ekzemen und Neurodermitis sowie Dysmenorrhöe. Bei niedrigeren Temperaturen können hiermit Wunden und Ekzeme sowie Neurodermitis behandelt werden.

Lavendelextrakt empfiehlt sich warm bei Erschöpfungszuständen, Nervosität, Schlafstörungen und vegetativer Dystonie.

Rosmarinextrakt empfiehlt sich warm bei Rheuma und Neuralgien und bei niedrigeren Temperaturen zur Anregung des Kreislaufes und zur Förderung der Durchblutung.

Schließlich kann dem Patienten zusätzlich während der Anwendung noch etwas ätherisches Öl zum Einatmen gegeben werden, wodurch weitere Steigerungen der Wirksamkeit oder bessere Verträglichkeit zu beobachten sind.

Die erfindungsgemäße Kombination von Pflanzenextrakten und/oder ätherischen Ölen mit einer Wärmepackung auf Basis von Fango empfiehlt sich schließlich in den Fällen, in denen eine hautreizende Wirkung des Fangos beobachtet wird und diese Wirkung gemildert werden soll.

Auf alle Fälle gestattet die erfindungsgemäße Packung, insbesondere die Wärmepackung auf Basis von Fango, heilende und lindernde Einzelwirkungen des Fangos einerseits und der Pflanzenextrakte und/oder ätherischen Öle andererseits zu kombinieren und mit synergistischen Effekten einzusetzen. Die Anwendung ist einfach und praktisch ohne Risiko, sofern die Patienten die zur Anwendung kommenden Pflanzenextrakte und/oder ätherischen Öle vertragen. Dies kann gegebenenfalls durch einfache Vorversuche mit dem Pflanzenextrakt und/oder ätherischem Öl allein festgestellt werden.

## Patentansprüche

1. Packung auf Basis von Fango und einem oder mehreren Pflanzenextrakten und/oder ätherischen Ölen, dadurch gekennzeichnet, dass Fango und Pflanzenextrakte und/oder ätherische Öle in vermischter Form vorliegen.

2. Packung gemäß Anspruch 1, dadurch gekennzeichnet, dass als Pflanzenextrakt und/oder ätherisches Öl Arnika, Rosmarin, Kamille, Rosskastanie, Lavendel oder Gemische derselben verwendet werden.

3. Verfahren zur Herstellung einer Packung auf Basis von Fango und einem oder mehreren Pflanzenextrakten und/oder ätherischen Ölen, dadurch gekennzeichnet, dass der gegebenenfalls bereits erwärmte, feuchte Fango mit dem oder den Pflanzenextrakten und/oder ätherischen Ölen vermischt und dann in üblicher Weise auf den Körper oder den Körperteil aufgebracht wird.

## Claims

1. A pack based on fango and one or more plant extracts and/or essential oils, characterized in that said fango and plant extracts and/or essential oils are present in a mixed form.

2. The pack according to claim 1, characterized in that arnica, rosemary, camomile, horse chestnut, lavender or mixtures thereof are used as said plant extract and/or essential oil.

3. A method for preparing a pack based on fango and one or more plant extracts and/or essential oils, characterized in that the wet fango, which optionally has already been heated, is mixed with said plant extract or extracts and/or essential oil or oils and theri applied to the body or part of the body in the usual way.

## Revendications

1. Enveloppement à base de fango et d'un ou plusieurs extraits de plantes et/ou huiles essentielles, caractérisé en ce que le fango et les extraits de plantes et/ou les huiles essentielles se présentent sous forme mélangée.

2. Enveloppement selon la revendication 1, caractérisé en ce qu'on utilise, en tant qu'extraits de plantes et/ou d'huiles essentielles, l'arnica, le romarin, la camomille, le marron d'Inde, la lavande ou leurs mélanges.

3. Procédé de fabrication d'un enveloppement à base de fango et d'un ou plusieurs extraits de plantes et/ou d'huiles essentielles, caractérisé en ce que le fango humide, éventuellement déjà chauffé, est mélangé avec le ou les extraits de plantes et/ou la ou les huiles essentielles, puis est appliqué d'une manière usuelle sur le corps ou sur la partie considérée du corps.
